# EUROPEAN PATENT APPLICATION

(11) **EP 4 023 567 A1**
(43) Date of publication of application: **06.07.2022**
(21) Application number: 20856009.4
(22) Date of filing: 20.08.2020
(51) Int. Cl.: B65D 85/00, A61L 9/01, A61L 9/03

(54) **FRAGRANCE DIFFUSION DEVICE AND FRAGRANCE DIFFUSION METHOD**

(30) Priority: 26.08.2019 JP 2019153484
(71) Applicant: Antbee, Inc., Ichikawa-shi, Chiba 272-0021 (JP)
(72) Inventor: NAGATO, Yasuyuki, Ichikawa-shi Chiba 272-0021 (JP)
(74) Representative: Schön, Christoph
(86) International application number: PCT/JP2020/031408
(87) International publication number: WO 2021/039568

(57) **Abstract**

[Technical Problem]

The problem of the present invention is to provide a fragrance diffuser which can diffuse a fragrance efficiently from a fragrance source with a calm atmosphere.

[Solution to Problem]

A fragrance diffuser 1 of the present invention is provided. The fragrance diffuser 1 comprises a Peltier element 10 comprising the first surface 11 and the second surface 12, a heat source installed part 20 to install a heat source 21 to heat the first surface 11, a fragrance source installed part 30 to install a fragrance source 31, a fan 40 to send the air to the fragrance source installed part 30, a motor 50 to drive the fan 40 and a circuit 60 to supply electric power to the motor 50 from the Peltier element 10.

## Description

### [Technical Field]

The present invention relates to a fragrance diffuser.

### [Background]

In late years, a tendency to improve how to spend time at home spreads out worldwide. The fragrance candle producing a fragrance relaxing a feeling attracts attention as an interior item.

The produced fragrance from an aroma candle tends to drift on the spot. Therefore, it was necessary to use the means that could diffuse the fragrance in a room together to utilize the fragrance efficiently.

A device using electric power such as batteries is known to diffuse the fragrance. For example, as a device to deodorize restrooms, etc., a device comprising the aromatic substance evaporating means consisting of an aromatic substance and a heating part to heat the aromatic substance exists as described in Patent Literature 1. The aromatic substance is evaporated by heating the aromatic substance with the heating part in such device.

As described in Patent Literature 2 and reference 3, a device diffusing fragrance using a Peltier element also exists. In such devices, it is described that evaporation of the fragrance is controlled by controlling a heating or a cooling by the Peltier element using electric power.

A fragrance source is evaporated by generating heat with electric power in these devices. However, the fragrance cannot be diffused widely only by evaporating the fragrance merely using heat in this way.

To diffuse the fragrance, means to diffuse using a fan is known conventionally. The fragrance can be diffused by using any fan. However, many of the fans used conventionally are very powerful fans and are not assumed to diffuse the fragrance gently.

On the other hand, the device producing the fragrance by the fragrance candle, etc. is often used in a calm atmosphere. When enjoying a relaxed atmosphere, a gentle light such as candles is preferred. Diffusing the fragrance by a fan rotating intensely ruins a calm atmosphere.

### [Citation list]

### [Patent Literature]

[Patent Literature 1] Japanese unexamined patent publication No. 2006-296962
[Patent Literature 2] Japanese KOKAI publication No. 2002-125751
[Patent Literature 3] Japanese KOKAI publication No. H8-336576

### [SUMMARY]

### [Technical Problem]

As described above, in the situation where the fragrance is used, the fragrance is enjoyed relaxedly with a calm atmosphere. In the conventional devices diffusing the fragrance, it is intended to diffuse the fragrance efficiently, and it is not assumed to create a calm atmosphere and to diffuse the fragrance gently. Therefore, the problem of the present invention is to provide the fragrance diffuser which can diffuse efficiently the fragrance from the fragrance source with a calm atmosphere.

### [Solution to Problem]

As a result of dedicated examination, the present inventor arrived at generating a voltage using Seebeck effect to rotate a fan slowly using said voltage by using the Peltier element and using the heat source of the candle. He arrived at to be able to generate the suitable voltage to rotate the fan gently by the calm heating of the candle and being able to create a calm atmosphere by the light of the candle and also to diffuse efficiently the fragrance from the fragrance source with the calm atmosphere.

Therefore, the present invention provides a fragrance diffuser comprising a Peltier element comprising the first surface and the second surface, a heat source installed part to install a heat source to heat the first surface, wherein the heat source produces flame and light, a fragrance source installed part to install a fragrance source, a fan to diffuse a fragrance from the fragrance source installed part, a motor to drive the fan and a circuit to supply electric power to the motor from the Peltier element.

The present invention also provides the fragrance diffuser, wherein the heat source is a candle or an alcohol lamp.

The present invention also provides the fragrance diffuser, wherein the fan comprises a fragrance source accommodating part as the fragrance source installed part.

The present invention also provides the fragrance diffuser, wherein the fragrance accommodating part comprises an evaporation device to evaporate a fragrance from the fragrance source, and the circuit supplies electric power to the motor and the evaporation device from the Peltier element.

The present invention also provides the fragrance diffuser, wherein the fan rotates at the rate of 0.5-5 times per second.

The present invention also provides a fragrance diffuser kit comprising the fragrance diffuser, a fragrance source and a heat source, wherein the fragrance source is installed in the fragrance source installed part of the fragrance diffuser and the heat source is installed in the heat source installed part of the fragrance diffuser.

### [Advantageous Effects of Invention]

The fragrance diffuser according to the embodiment can diffuse a fragrance efficiently.

### [Brief Description of Drawings]

Figure 1 is a schematic perspective view showing an example of the fragrance diffuser kit of the present invention.
Figure 2 is a front view of an example of the fragrance diffuser of the present invention.
Figure 3 is a right side view of the fragrance diffuser of Figure 2.
Figure 4 is a plane view of the fragrance diffuser of Figure 2.
Figure 5 is a left side view of the fragrance diffuser of Figure 2.
Figure 6 is a rear view of the fragrance diffuser of Figure 2.
Figure 7 is a bottom view of the fragrance diffuser of Figure 2.
Figure 8 is a schematic front diagram showing an example of the fragrance diffuser kit of the present invention.
Figure 9 is a schematic diagram showing an example of the fragrance source installed part of the fragrance diffuser of the present invention.
Figure 10 is a schematic diagram showing an example of the fragrance source installed part of the fragrance diffuser of the present invention.
Figure 11 is a schematic diagram showing an example of the fragrance source installed part of the fragrance diffuser of the present invention.
Figure 12 is a schematic diagram of the Peltier element of an example that the fragrance diffuser according to the embodiment can comprise.
Figure 13 is a perspective diagram of the motor of an example that the fragrance diffuser according to the embodiment can comprise.
Figure 14 is a circuit diagram of the fragrance diffuser of an example according to the embodiment.
Figure 15 is a graph showing the relationship between the distance from the heat source to the first surface of the Peltier element and the rotational velocity of the fan.

### [Description of Embodyments]

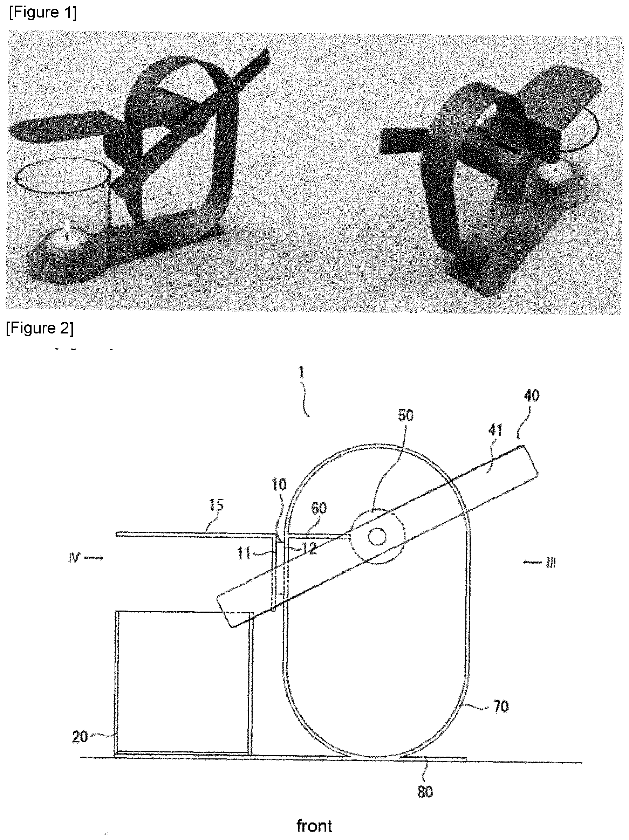

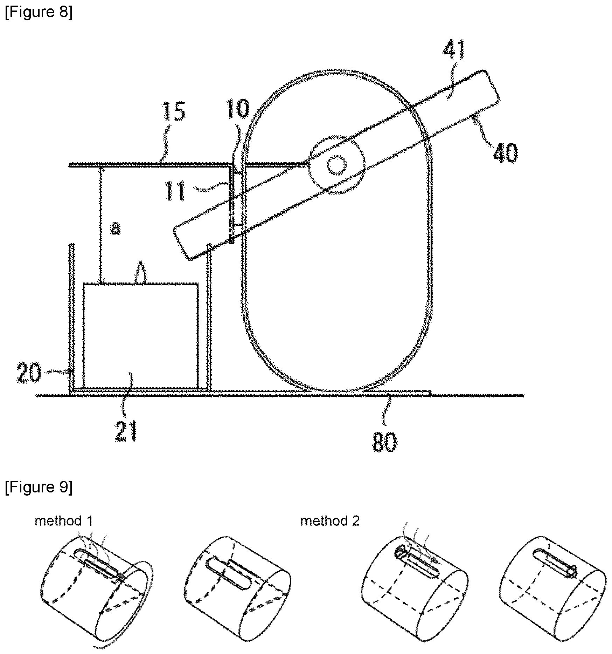

Figure 1 shows a perspective view of an example of the fragrance diffuser of the present invention. Figure 2-7 also show six-views about an example of the fragrance diffuser of the present invention. Figure 8 also shows the fragrance diffuser kit comprising the fragrance diffuser of the present invention. The fragrance diffuser of the present invention is described by taking one embodiment thereof as an example with reference to the drawings as follows. In the drawings, the broken lines show the parts which do not appear.

The present fragrance diffuser 1 comprises a Peltier element 10 comprising the first surface 11 and the second surface 12, a heat source installed part 20 to install a heat source 21 to heat the first surface 11, a fragrance source installed part 30 to install a fragrance source 31, a fan 40 to diffuse a fragrance from the fragrance source installed part 30, a motor 50 to drive the fan 40 and a circuit 60 to supply electric power to the motor 50 from the Peltier element 10.

A Peltier element is a semiconductor element which can control cooling and heating of said metal by transferring heat from one metal to another metal by applying direct current to the junction of two kinds of metal.

In all materials, a potential difference occurs between the both ends by providing the temperature difference at the both ends. This phenomenon is called Seebeck effect. The Peltier element can also generate the voltage by providing the temperature difference between two kinds of metal. The Peltier element is used as a thermoelectric conversion element widely since the electromotive force due to this Seebeck effect is large.

The Peltier element 10 that the fragrance diffuser 1 of the present invention comprises includes the first surface 11 and the second surface 12. The first surface 11 and the second surface 12 is equivalent to the both sides of the Peltier element 10. In the fragrance diffuser 1 of the present invention, the first surface 11 can be heated by the heat source 21 installed in the heat source installed part 20.

On the other hand, the second surface 12 of the Peltier element 10 is not heated by the heat source 21 installed in the heat source installed part 20. Thus, the temperature difference can be caused between the first surface 11 and the second surface 12 at either side of the Peltier element 10. Due to this temperature difference, the Peltier element shows Seebeck effect. Electric power is supplied to the motor 50 as direct current from the generated voltage through the circuit 60. The motor 50 can drive the fan 40 by this electric power. The fan 40 can diffuse the fragrance from the fragrance source 31 installed in the fragrance source installed part 30 by rotating.

Furthermore, the fan 40 can send the air to the second surface 12 of the Peltier element 10. By the air blow from the fan 40, the second surface 21 can be cooled. Thereby, the temperature difference between the first surface 11 and the second surface 12 of the Peltier element 10 can be made greater. As a result, the Peltier element 10 can generate the greater voltage to supply electric power to the motor 50. The motor 50 which was supplied electric power can also provide stably and greater rotational force to the fan 40. Thereby, the air blow by the fan 40 can be increased more and the fragrance can be diffused more efficiently.

Thus, the fragrance diffuser 1 according to the first embodiment can diffuse the fragrance efficiently.

According to the second embodiment, the fragrance diffuser kit 100 comprising the fragrance diffuser 1 according to the first embodiment, the fragrance source 31 and the heat source 21 is also provided. The fragrance source 31 is accommodated in the fragrance source installed part 30 of the fragrance diffuser 1. The heat source 21 is installed in the heat source installed part 20 of the fragrance diffuser 1.

The fragrance diffuser kit 100 according to the second embodiment can diffuse the fragrance efficiently by heating the fragrance diffuser 1 according to the first embodiment by the heat source 21.

Then, the fragrance diffuser 1 according to the first embodiment and the fragrance diffuser kit 100 according to the second embodiment are described in more detail.

The fragrance diffuser 1 according to the first embodiment comprises the Peltier element 10, the heat source installed part 20, the fragrance source installed part 30, the fan 40, the motor 50 and the circuit 60. The fragrance diffuser kit according to the second embodiment comprises the fragrance diffuser 1 according to the first embodiment, the heat source 21 and the fragrance source 31.

The Peltier element 10 comprises the first surface 11 and the second surface 12. It is preferable that the first surface 11 and the second surface 12 made of the material with low specific heat, respectively. The materials with low specific heat have a large amount of rise in temperature by heating from the heat source. The materials with low specific heat also have a large amount of decrease in temperature by the air blow from the fan. For example, the materials with low specific heat are metal or alloy. Metal or alloy includes, for example, copper, silver, zinc, aluminum, aluminum alloy and stainless steel, etc. Because aluminum or aluminum alloy is lightweight, they are more preferable. The material constituting the first surface 11 may be the same as the material constituting the second surface 12 or may be different material.

The Peltier element 10 comprises an N-polarity (N-type) semiconductor and a P-polarity (P-type) semiconductor. The N-polarity semiconductor is sandwiched between the first surface 11 and the second surface 12. Similarly, the P-polarity semiconductor is sandwiched between the first surface 11 and the second surface 12. The Peltier element 10 may comprise one N-polarity semiconductor or may comprise a plurality of N-polarity semiconductors. The Peltier element 10 may also comprise one P-polarity semiconductor or may comprise a plurality of N-polarity semiconductors.

The N-polarity semiconductor comprises the high purity semiconductor as the main component and a very small amount of quinquevalent element as impurities. The high purity semiconductor is typically silicon (Si). The quinquevalent element is typically phosphorus (P).

The P-polarity semiconductor comprises the high purity semiconductor as the main component and a very small amount of trivalent element as impurities. The high purity semiconductor is typically silicon (Si). The trivalent element is typically boron (B).

The Peltier element 10 can have, for example, the structure of the π type. The Peltier element having the structure of the π type comprises, for example, one first metal plate as the first surface, two second metal plates as the second surface, one N-type semiconductor and one P-type semiconductor. The N-type semiconductor is sandwiched between the first metal plate and one second metal plate. The P-type semiconductor is sandwiched between the first metal plate and the other second metal plate. The Peltier element 10 can also have a structure that above-mentioned π type structures are arranged in series. The Peltier element 10 comprising the structure that a plurality of π type structures is arranged in series can show the larger electromotive force.

For example, one second metal plate of the Peltier element having π type structure can be connected to the positive terminal of the motor through the circuit. The other second metal plate of the Peltier element can also be connected to the negative terminal of the motor through the circuit.

Note that the details of the Peltier element 10 are not particularly limited as long as it functions as a Peltier element.

The first surface 11 of the Peltier element 10 may be also heated by heat source 21 directly or may be heated indirectly. For example, as shown in Figures 1-7, the first surface 11 can be heated through the heating part 15. For example, the heating part 15 and the first surface 11 made of metal can be joined so that heat conduction is possible, and the first surface 11 may be heated by heating the heating part 15 and thereby transferring heat from the heating part 15 to the first surface 11.

Similarly, the second surface 12 of the Peltier element 10 may be cooled directly or may be also cooled indirectly by the air blow from the fan 40. For example, as shown in Figures 1-7, the second surface 21 can be cooled through the fan support 70. For example, the fan support 70 and the second surface 21 made of metal can be joined so that heat conduction is possible, and the second surface 21 may be cooled by cooling the fan support 70 by the air blow from the fan 40 and transferring heat thereof to the second surface 21 from the fan support 70. Also, the second surface 21 of the present invention may not be cooled by the air blow from the fan 40. The fragrance generator 1 of the present invention can heat the first surface 11 by the heat source 21 sufficiently. Therefore, even if the second surface 21 is not cooled, the enough temperature difference is caused between the first surface 11 and the second surface 12, and the Peltier element 10 can supply electric power.

The heat source installed part 20 is constituted to accommodate the heat source 21. The heat source installed part 20 can adopt various forms depending on a kind of the heat source 21 installed. The heat source 21 can be any heat source which can heat the first surface 11 of the Peltier element 10. For example, the heat source 21 can be a heat source producing flame and light such as a candle, an alcohol lamp, a gas burner, a solid fuel, a gas stove, Chakkaman (gas lighter), a bonfire or charcoal. The heat source 21 can be preferably a candle or an alcohol lamp. When the heat source is a heat source producing flame such as candles, the heat source installed part 20 is a folder that a candle and the like can be placed, for example. For example, it can be the folder made of transparent materials which allow the visible light to pass through. By using such folder, a shimmering flame such as candles can be recognized visually from the outside. The shimmering flame such as candles can give an ease to humans visually. The shimmering flame such as candles can also provide a calm atmosphere. The residual quantity can be also checked even if the heat source such as candles is not taken out of the folder if it is the folder made of transparent materials which allow the visible light to pass through. The heat source installed part 20 is also installed depending on desired heat source 21 such as a candle or an alcohol lamp so that the heating part 15 can be heated to desired temperature. The heat sources such as candles vary in size of flame depending on size. Therefore, in consideration of heat quantity, etc. of the heat source 21, the heat source installed part 20 can be installed in the position that the heating part 15 can be heated at the temperature which can supply electric power to rotate the fan 40 at desired rotation number. For example, when the Peltier element generates the large voltage because the heat source produces large flame, the temperature of the heating part can be regulated by installing the heat source installed part keeping the heat source away from the heating part.

Alternatively, the heat source 21 may be an electric heater. In this embodiment, the heat source installed part 20 comprises an electric heater and an electric power supply folder to install an electric power supply. The electric power supply can supply electric power to the electric heater.

It is preferable that the heat source installed part 20 is made of the heat-resistant materials. In the embodiment wherein the heat source is also a candle, it is preferable that the heat source installed part 20 is made of fireproof materials.

The fragrance source installed part 30 is constituted to install the fragrance source 31. Therefore the fragrance source installed part 30 can adopt various forms depending on a kind of the fragrance source 31.

The fragrance source installed part 30 can be installed in the position where the fragrance can be diffused by the wind occurred by the fan 40. For example, the fragrance source installed part 30 can diffuse the fragrance with the wind taken in and exhausted by being installed on the intake side of the fan 40. On the contrary, the fragrance source installed part 30 can diffuse the fragrance with exhausted wind by being installed on the exhaust side of the fan 40.

In another embodiment, the fragrance source installed part 30 can be the form that can regulate the quantity of the evaporated fragrance. For example, as shown in Figure 9, the size of the opening of the fragrance source installed part 30 may be adjustable. The quantity of the fragrance released from the opening can be regulated by being able to open and close the size of the opening of the fragrance source installed part 30 by a rotating mechanism or a slide mechanism.

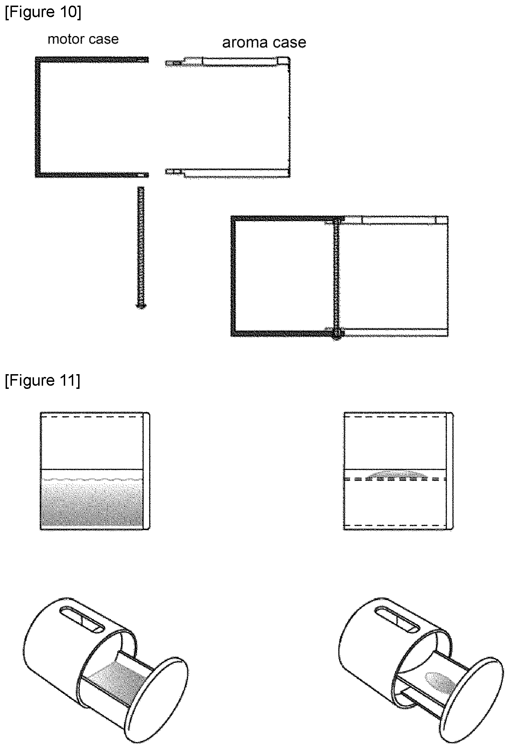

In another embodiment, the fragrance source installed part 30 may be installed integrally with the fan 40 or the motor 50. For example, as shown in Figure 10, the fragrance source installed part 30 is may be installed integrally with a motor case. The motor 50 is accommodated in the motor case and the fragrance source 31 is accommodated in the fragrance source installed part 30.

The fragrance source installed part 30 can accommodate any quantity of the fragrance source 31. For example, as shown in the left side of Figure 11, a large quantity of the fragrance source 31 may be able to be accommodated. In this case, the fragrance can be diffused for a long term. As shown in the right side of Figure 11, it may be also the size that can accommodate the fragrance source 31 for one time. In Figure 11, the upper section shows the cross-sectional view in a condition where the fragrance source 31 is accommodated, and the lower section shows the condition where the accommodating part of the fragrance source 31 is drawn in the fragrance source installed part 30.

In another embodiment, the fragrance source installed part 30 can further comprise the evaporation device 33 to evaporate the fragrance from the fragrance source. That is, the evaporation device 33 is constituted to evaporate the fragrance from the fragrance source 31. For example, the evaporation device 33 can be a heater. The heater applies heat to the fragrance source 31 and can evaporate the fragrance included in the fragrance source 31. In this embodiment, the Peltier element 10 can supply electric power to the evaporation device 33, for example, the heater through the circuit 60.

The fragrance source 31 is not particularly limited as long as it is the form that can diffuse the fragrance by the fan 40. For example, the fragrance source 31 can comprise the container accommodating the absorbent which absorbed the fragrant substance. This container may comprise the vent 32. The fragrance source 31 can take the air in from the vent 32 and dissipate the fragrance absorbed in the absorbent from the vent 32. The container may comprise the first surface provided with the first vent and the second surface provided with the second vent. The first surface and the second surface are the different surfaces of the container with respect to one another. In this preferred embodiment, the air taken in from the first vent allows the fragrance to be dissipated efficiently from the second vent. Alternatively, the air taken in from the second vent allows the fragrance to be dissipated efficiently from the first vent. It is desirable that the first surface and the second surface are approximately parallel each other. It is desirable that the fragrance source installed part 30 is constituted to install the fragrance source without blocking the first and the second vent of the container of the fragrance source 31.

The fragrance that the fragrance source 31 comprises is not particularly limited. For example, the fragrance source 31 can be any aromatic substances such as the fragrance oil. In the fragrance source 31, the fragrance source 31 may be a liquid, may be a solid or may be adsorbed in a solid. It may be the fragrance source 31 that the fragrance is absorbed in the absorbent. Examples of the absorbent include cotton, absorption beads, however are not particularly limited.

The fan 40 is installed to be able to send the air to diffuse the fragrance from the fragrance source installed part 30 of the Peltier element 10, due to driving force transmitted from the motor 50. The embodiment of the fan 40 is not particularly limited as long as it is driven by the motor 50. The fan 40 can have, for example, the shaft mounting part 42 and the blade 41. The shaft mounting part 42 comprises a groove to intrude the shaft of the motor 50. It is preferable that the blade 41 is point symmetry about the shaft mounting part 42. The number and the form of blades are not particularly limited.

For example, the blade may be installed with the fragrance source installed part 30. In this embodiment, the fragrance source installed part 30 can be the concave portion provided with the blade 41. This concave portion is constituted to accommodate the fragrance source 31. Also, in this embodiment, the blade 41 may further comprises a stopper to fix the fragrance source 31 accommodated in the concave portion. In this embodiment, the fragrance from the fragrance source 31 accommodated in the fragrance source installed part 30 of the blade 41 can receive the wind by rotating the fan 40. That is, in this embodiment, the fragrance source installed part 30 of the blade 41 can be diffused due to the air blow by the blade 41.

The fan 40 can be rotated at any rate. For example, the fan 40 can be rotated at the rate of 0.2-10 times per second, namely 30-600 times per minute. The fan 40 rotating at this speed, namely the fan 40 that rotational velocity of the blade 41 is within this range can diffuse the fragrance sufficiently. The fragrance generator 1 of the present invention can also diffuse the fragrance from the fragrance source efficiently with a calm atmosphere. That is, by rotating the fan 40 at the rate of 0.2-10 times per second, the fragrance can be diffused efficiently and the calm atmosphere is not ruined. The more preferable rotational velocity of the blade is 0.5-8 times per second and 0.8-8 times per second, for example, 1-5 times per second and 2-4 times per second.

The motor 50 is constituted to obtain electric power from the Peltier element 10 to transmit the driving force to the fan 40. The motor 50 is not particularly limited as long as it can obtain electric power from the Peltier element 10 to transmit the driving force to the fan 40. The motor 50 comprises the shaft which is mounted, for example, to the shaft mounting part of the fan 40. The motor 50 can also be installed integrally with the fragrance source accommodating part 30 described above.

The circuit 60 is a circuit controlling electric power supply from the Peltier element 10 to the motor 50. If electric power supply from the Peltier element 10 to the motor 50 can be controlled, the circuit 60 can be any circuit and is not particularly limited. The circuit 60 may also supply electric power to other components, for example, the evaporation device in addition to electric power supply from the Peltier element 10 to the motor 50. While the circuit 60 supplies electric power necessary to rotate the fan to the motor 50 based on the voltage generated from the Peltier element 10, it can supply remaining electric power to other components such as the evaporation device.

In one embodiment, the fragrance diffuser can comprise the input terminal from an external power supply.

Then, the specific example of the fragrance diffuser and the fragrance diffuser kit of the present invention are described in more detail with reference to the drawings. Note that the example described below is intended to merely exemplify one embodiment of the present invention and does not intend to limit the present invention.

Note that, in drawings, similar components have similar reference numbers. In the following description, overlapped descriptions are also omitted as for similar componentry.

At first, with reference to Figure 1, the example of the fragrance diffuser kit of the present invention is described. Figure 1 is a schematic perspective diagram showing an example of the fragrance diffuser kit of the present invention. The fragrance diffuser kit 100 shown in Figure 1 comprises the fragrance diffuser 1, the heat source 21 and the fragrance source 31. The fragrance diffuser 1 comprises the base 80 as the bottom surface. On the base 80, the cylindrical heat source installed part 20 formed of transparent materials which allow the visible light to pass through is arranged. On the base 80, the fan support 70 is also comprised at the position away from the heat source installed part 20. Above the heat source installed part 20, the heating part 15 is arranged at the position where it can be heated by the candle as the heat source 21. The fragrance diffuser 1 also comprises the Peltier element 10 between the heat source installed part 20 and the fan support 70. The Peltier element 10 have the first surface 11 facing to the heat source installed part 20 and the second surface 12 facing to the fan support 70. In the Peltier element 10, the N-polarity semiconductor and the P-polarity semiconductor which are not illustrated are arranged between the first surface 11 and the second surface 12. The fan support 70 comprises a holding part of the circuit 60 in a part thereof. The fan 40 and the motor 50 are also comprised in the inside of elliptic fan support 70 through the holding part of the circuit 60.

The motor 50 comprises the shaft which is not illustrated. The shaft of the motor 50 is mounted to the shaft mounting part 42 of the fan 40. The fan 40 comprises one piece of the blade 41 having the shape that is point symmetric to the shaft mounting part 42. The fragrance source installed part 30 is formed integrally with a case of the motor 50, and the fan 40 is also formed integrally through the motor 50. The motor 50 is connected to the Peltier element 10 electrically through the circuit which is not illustrated.

In the fragrance diffuser kit 100 shown in Figure 1, the heat source 21 installed in the heat source installed part 20 is a candle. The flame of the candle 21 can heat the heating part 15 connected to the first surface 11 of the Peltier element 10 so that heat conduction is possible. The flame of the candle 21 is adjusted not to touch the heating part 15.

By conducting heat to the first surface 11 since the heat source 21 heat the heating part 15, the temperature of the first surface 11 increases and thereby the temperature difference between the first surface 11 and the second surface 12 is produced. The Peltier element 10 generates the voltage using this temperature difference. The electric power generated is supplied to the motor 50 as direct current through the circuit.

The motor 50 can rotate the blade 41 of the fan 40 through the shaft by supplied electric power. Due to air intake by the fan 40 by rotating the blade 41, the second surface 12 of the Peltier element 10 can be cooled. The temperature of the second surface 12 decreases by air when air intake and the temperature difference between the first surface 11 and the second surface 12 can be increased.

The fragrance from the fragrance source installed part 30 formed integrally with the case of the motor 50 is also taken in together by the rotation of the blade 41. The fragrance source installed part 30 accommodates the fragrance source which is not illustrated. The fragrance source accommodating part also comprises the vent which is not illustrated. Therefore, the air taken in by the fan 40 enters into the fragrance source through the vent of the fragrance source installed part 30 and is exhausted with the fragrance from the vent. The fragrance is diffused by exhausting the air with the fragrance by the fan 40.

Then, referring to Figure 2-7, the fragrance diffuser of an example according to the embodiment is described in more detail.

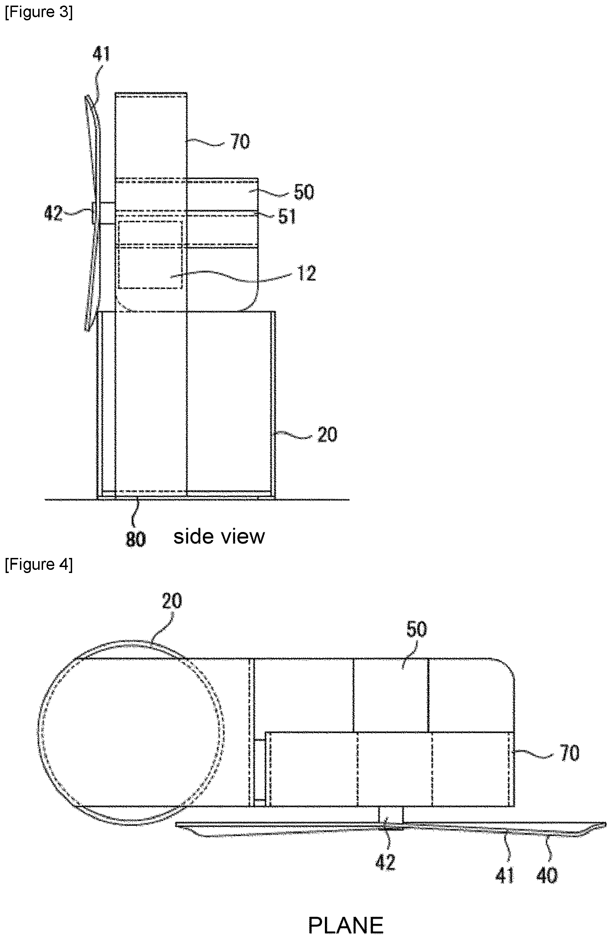

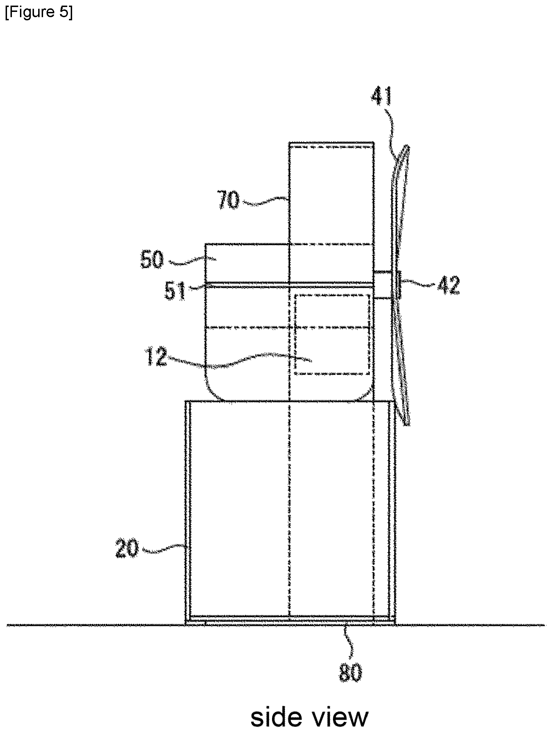

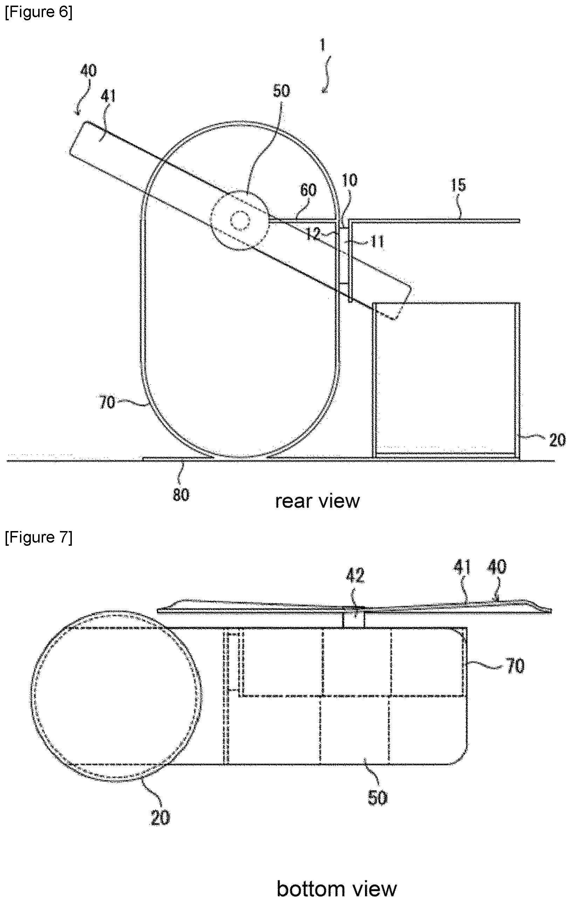

Figure 2 is a front view of an example of the fragrance diffuser of the present invention. Figure 3 is a right side view of the fragrance diffuser of Figure 2. That is, Figure 3 is a side view seen from viewpoint III of Figure 2. Figure 4 is a plane view of the fragrance diffuser of Figure 2. Figure 5 is a left side view of the fragrance diffuser of Figure 2. That is, Figure 5 is a side view seen from viewpoint V of Figure 2. Figure 6 is a rear view of the fragrance diffuser of Figure 2. Figure 7 is a bottom view of the fragrance diffuser of Figure 2.

The fragrance diffuser 1 shown in Figures 2-7 comprises the base 80 as the bottom surface. As shown in Figure 2, the heat source installed part 20 and the fan support 70 are installed on the base 80. The heat source installed part 20 and the fan support 70 are arranged so as to sandwich the Peltier element 10.

The Peltier element 10 comprises the first surface 11, the second surface 12, the N-polarity semiconductor element 13 sandwiched between them and the P-polarity semiconductor element which is not illustrated. The first surface 11 is connected to heating part 15 located above of the heat source installed part 20 so that heat conduction is possible. The second surface 12 contacts with a part of the fan support 70 so that heat conduction is possible.

The fan support 70 supports the fan 40 and the motor 50. The motor 50 comprises the shaft 51. The shaft 51 is mounted to the shaft mounting part 42 of the fan 40. The fan support 70 comprises the circuit 60 in the holding unit. The circuit 60 installed in the fan support 70 holding part connects the motor 50 to the Peltier element 10 electrically.

Figure 8 is a schematic front view of an example of the fragrance diffuser kit of the present invention comprising the fragrance diffuser 1 and the heat source 21 shown in Figures 2-7 and the fragrance source which is not illustrated. The heat source 21 is a candle. The fragrance source installed part 30 shown in Figure 8 can efficiently diffuse the fragrance for the same reason as the fragrance source installed part 30 described with reference to Figure 1 can efficiently diffuse the fragrance.

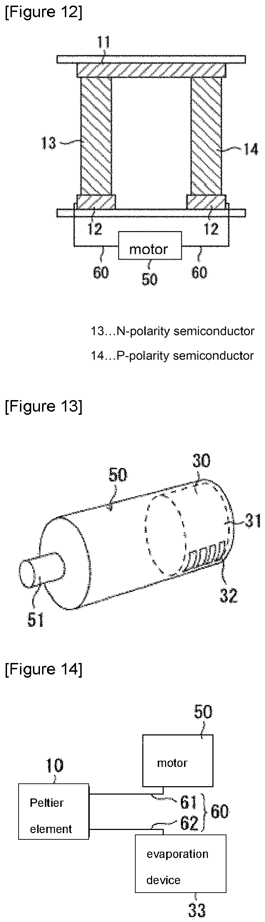

Then, an example of the Peltier element that the fragrance diffuser 1 of the present invention can comprise is described referring to Figure 12.

Figure12 is a schematic view showing an example of the Peltier element that the fragrance diffuser according to the embodiment can comprise.

The Peltier element 10 shown in Figure 12 comprises the first surface 11 in one and the second surface 12 in the other. Also, the Peltier element 10 further comprises the N-polarity semiconductor 13 sandwiched between the first surface 11 and one second surface and the P-polarity semiconductor 14 sandwiched between the first surface 11 and the other second surface. The Peltier element 10 shown in Figure 12 is the Peltier element having π type structure.

The semiconductor contacting with the second surface 12 is connected to the circuit 60. Also, through the circuit 60, it is connected to the motor 50 electrically.

As described above, the temperature difference is caused between the first surface 11 and the second surface 12 since the first surface 11 is heated by the heat source, and thereby the Peltier element 10 can generate electric power. The generated electric power is transferred to the motor 50 through the circuit 60.

Then, the example of the motor that the fragrance diffuser of the present invention can comprise is described referring to Figure 13.

Figure 13 is a perspective diagram of the motor of an example that the fragrance diffuser according to the embodiment can comprise. The motor 50 shown in Figure 13 comprises the shaft 51. This shaft 51 can be mounted to the shaft mounting part of the fan comprised in the fragrance diffuser. The motor 50 shown in Figure 13 further comprises the fragrance source installed part 30 formed integrally with the motor case. The fragrance source installed part 30 accommodates the fragrance source 31.

Figure 14 is a circuit diagram in an example of the fragrance diffuser 1 of the present invention. Figure 14 is a circuit diagram related to the embodiment wherein the fragrance source installed part 30 comprises the evaporation device. The circuit 60 shown in Figure 14 comprises the circuit 61 and the circuit 62. The circuit 61 connects the motor 50 to the Peltier element 10 electrically. The circuit 62 connects the evaporation device 33 to the Peltier element 10 electrically. As shown in Figure 14, in embodiment wherein the motor 50 comprises the fragrance source installed part 30 comprising the evaporation device, the fragrance diffuser 1 can be connected to the circuit 61 to the motor 50 and to the circuit 62 to the evaporation device diverging from the circuit 60 connecting from the Peltier element 10 to the motor 50.

The fragrance diffuser of the present invention described above can efficiently diffuse the fragrance from the fragrance source by rotating the fan using the Peltier element. By using the candle as a heat source and rotating the fan slowly, a calmer atmosphere can be also created not only by the effect of the diffused fragrance but also by the movement of the flame of the candle and the fan.

### [Examples]

Then, the example is described. However, the example is intended to describe as the specific example of the embodiments and does not limit the present invention.

### [Example 1] In Example 1, the fragrance diffuser kit 100 having a structure same as the one illustrated schematically in Figure 8 was created.

As the heat source 21, a candle was used. As the heating part 15 contacting with the first surface 11 of the Peltier element 10 and the fan support contacting with the second surface 12, the aluminum plates were used.

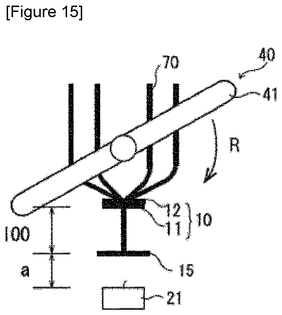

In Example 1, the distance "a" from the heat source 21 to the first surface 11 of the Peltier element 10 shown in Figure 8 was set to 5mm. The distance between the first surface 11 and the second surface 12 of the Peltier element 10 was also set to 100mm. A relationship of the distance a and the distance between the first surface 11 and the second surface 12 is shown schematically in Figure 15. The candle used was a cylindrical candle of 3.7cm in diameter and 1.9cm in height.

In the fragrance diffuser kit 100 of Example 1, approximately 3 minutes later after igniting the candle, the blade 41 started to rotate. 30 minutes after igniting the candle, the blade 41 rotated stably. The rotation number of the blade 41 of the fan 40 was as follows.

**[Table 1]**

| 3 minutes later after igniting the candle | start of rotation |
|---|---|
| 30 minutes later after igniting the candle | 3 times/s of rotational velocity of the blade |
| 60 minutes later after igniting the candle | 2.5 times/s of rotational velocity of the blade |
| 120 minutes later after igniting the candle | 3.5 times/s of rotational velocity of the blade |
| 280 minutes later after igniting the candle | 2.4 times/s of rotational velocity of the blade |
| 240 minutes later after igniting the candle | 2.3 times/s of rotational velocity of the blade |
| 300 minutes later after igniting the candle | 2.1 times/s of rotational velocity of the blade |

A rotation of the blade 41 started in around 3 minutes after the candle was ignited. After 30 minutes of the ignition, it rotated at 3 times/second of rotational velocity. After 300 minutes of the ignition, it rotated at 2.1 times/second of rotational velocity. Because the height of the flame changes as the remaining quantity of the wax of the candle decreases, the rotation number of the blade decreased slightly with the combustion of the candle. Due to heating by the candle, the blade was able to be rotated stably at 2-3 rotations/second of rotational velocity during 300 minutes after the ignition. While the candle was burnt, the fragrance was able to be diffused sufficiently by rotating at 1-5 rotations/second of rotational velocity desirable as the fragrance diffuser of the present invention.

By regulating the height and the size of the heat source (the candle in the example) and the shape and height of the heat source installed part, etc., it was also shown to be able to regulate the rotation number of the blade so that the desirable rotation number was obtained. As shown in Figure 15, the rotational velocity R can be regulated by regulating the distance a from the heat source 21 to the heating part 15.

When burning the candle in the above fragrance diffuser indoors with ceiling height of 3m and 82m² in area, the fragrance was able to be felt right after rotating the fan. On the other hand, when the fan was not rotated, the distance to feel the fragrance was apparently short in comparison with the time when the fan was rotated. The fragrance diffuser of the present invention was able to diffuse the fragrance sufficiently by rotating the blade stably at a rotational velocity of 2-3 rotations/second.

According to one or more embodiments described above, the fragrance diffuser is provided. This fragrance diffuser comprises a Peltier element comprising the first surface and the second surface, a heat source installed part to install a heat source to heat the first surface, a fragrance source installed part to install a fragrance source, a fan to send the air to the second surface and the fragrance source installed part, a motor to drive the fan and a circuit to supply electric power to the motor from the Peltier element. In this fragrance diffuser, the fan is driven by electric power supplied by the Peltier element and the fragrance can be diffused efficiently from the fragrance source.

The present invention is not limited to each embodiment described above, and various kinds of modifications are possible within the scope of claims, and the embodiments obtained by combining appropriately the technical means disclosed in different embodiments, respectively, are also included in the technical scope of the present invention.

### [Industrial Applicability]

The fragrance diffuser according to the embodiment can be used as an item enjoying the fragrance.

### [Reference Signs List]

1...fragrance generator, 10...Peltier element, 11...first surface, 12...second surface, 13...N-polarity semiconductor, 14...P-polarity semiconductor, 15...heating unit, 20...heat source installed part, 21...heat source, 30...fragrance source installed part (fragrance source accommodating unit), 31...fragrance source, 32...vent, 33...evaporation device, 40...fan, 41...blade, 42...shaft mounting unit, 50...motor, 51...shaft, 60, 61 and 62...circuit, 70...fan support, 80...base, 100...fragrance diffuser kit.

## Claims

1. A fragrance diffuser comprising:
a Peltier element comprising the first surface and the second surface;
a heat source installed part to install a heat source to heat the first surface, wherein the heat source is the heat source producing flame or light;
a fragrance source installed part to install a fragrance source;
a fan to diffuse a fragrance from the fragrance source installed part;
a motor to drive the fan; and
a circuit to supply electric power to the motor from the Peltier element.

2. The fragrance diffuser according to claim 1, wherein the heat source is a candle or an alcohol lamp.

3. The fragrance diffuser according to claim 1, wherein the fan is formed integrally with the fragrance source installed part.

4. The fragrance diffuser according to claim 3, wherein:
the fragrance accommodating part comprises an evaporation device to evaporate a fragrance from the fragrance source; and
the circuit supplies electric power to the motor and the evaporation device from the Peltier element.

5. The fragrance diffuser, wherein the fan rotates at the rate of 0.5-5 times per second.

6. A fragrance diffuser kit comprising:
the fragrance diffuser according to claim 1;
the fragrance source; and
the heat source;
wherein
the fragrance source is installed in the fragrance source installed part of the fragrance diffuser; and
the heat source is installed in the heat source installed part of the fragrance diffuser.
